# EUROPEAN PATENT SPECIFICATION

(11) **EP 4 551 050 B1**
(45) Date of publication and mention of the grant of the patent: **02.09.2026**
(21) Application number: 23738736.0
(22) Date of filing: 05.07.2023
(51) Int. Cl.: A24F 40/05, A24F 40/20, A61M 15/00, A24F 42/20, A24F 42/60, A61M 15/06

(54) **INHALER ARTICLE WITH POWDER LEAK PROTECTION**
INHALATORARTIKEL MIT PULVERLECKSCHUTZ
ARTICLE INHALATEUR AVEC PROTECTION CONTRE LES FUITES DE POUDRE

(30) Priority: 05.07.2022 EP 22183053
(43) Date of publication of application: 14.05.2025
(73) Proprietor: Philip Morris Products S.A., 2000 Neuchâtel (CH)
(72) Inventor: CAMPITELLI, Gennaro, 2000 Neuchâtel (CH); D'AMBROGI, Valerio, 20121 Milano (IT)
(74) Representative: Abitz & Partner
(86) International application number: PCT/EP2023/068595
(87) International publication number: WO 2024/008817

(56) References cited:
- WO-A1-2022/123487
- WO-A1-2022/130090
- US-A1- 2011 277 752
- US-A1- 2021 008 306

## Description

The present invention relates to an inhaler article. The disclosure further relates to an inhaler article holder and to an inhaler system comprising an inhaler article and an inhaler article holder.

It is known to provide an inhaler system comprising an inhaler article holder that may be combined with an inhaler article containing a capsule. The inhaler article holder may be used to activate the inhaler article by puncturing the capsule with a piercing element. Particles may be released from the capsule upon drawing or creating an airflow around the pierced capsule. The inhaler system thus delivers the dry powder particles to a consumer. The inhaler article holder is separate from the inhaler article, but the consumer utilizes both the inhaler article and the inhaler article holder while consuming the dry powder particles released within the inhaler article. A plurality of these inhaler articles may be combined with an inhaler article holder to form a system or kit. A single inhaler article holder may be utilized on multiple inhaler articles to activate (puncture or pierce) a capsule contained within each inhaler article.

The following documents disclosed various inhaler articles WO 2022/130090 A1, US 2021/008306 A1, US 2011/277752 A1 and WO 2022/123487 A1.

It would be desirable to have an inhaler article with powder leakage protection. It would be desirable to have an inhaler article with reliable powder delivery. It would be desirable to have an inhaler system with powder leakage protection.

The invention is defined in the appended claims.

According to an embodiment of the disclosure there is provided an inhaler article comprising an upstream compartment. A pierceable capsule containing powder is arranged in the upstream compartment. The inhaler article further comprises a downstream compartment. The downstream compartment is fluidly connected with the upstream compartment via an air inlet arranged in a proximal end wall of the downstream compartment. The downstream compartment comprises an air outlet arranged in a distal portion of the downstream compartment. An entrance to the air outlet is arranged proximally distanced from a distal end wall of the downstream compartment.

According to an embodiment of the disclosure there is provided an inhaler article that may comprise an upstream compartment. A pierceable capsule containing powder may be arranged in the upstream compartment. The inhaler article may further comprise a downstream compartment. The downstream compartment may be fluidly connected with the upstream compartment via an air inlet arranged in a proximal end wall of the downstream compartment. The downstream compartment may comprise an air outlet arranged in a distal portion of the downstream compartment. An entrance to the air outlet may be arranged proximally distanced from a distal end wall of the downstream compartment.

Arranging the air outlet proximally distanced from the distal end wall of the downstream compartment, powder is prevented from leaking out of the air outlet and instead is collected between the distal end wall of the downstream compartment and the air outlet. Leakage of powder may be particularly problematic if the user rotates the inhaler article during use or between uses and the arrangement of the air outlet described herein solves this leakage issue or at least reduces leakage of powder in this situation.

The air outlet may comprise an outlet extension axis. An outlet angle of at least 5°, preferably of at least 10°, more preferably of at least 15°, most preferably of at least 20°, may be provided between the outlet extension axis and a longitudinal axis of the inhaler article.

In other words, the air outlet may be tilted. The air outlet may be tilted away from the longitudinal axis of the inhaler article. A tilting of the air outlet may reduce leakage of powder through the air outlet. Additionally, tilting of the air outlet may create swirling vortices in the airflow which may improve the delivery of the powder to the consumer.

The air outlet may be tilted such that air flowing out of the outlet predominantly flows with an angle with respect to the longitudinal axis of the inhaler article. This may be beneficial as the air carrying the powder may be directed in a desired way for example predominantly towards a tongue or a throat of a user.

The air inlet may comprise an inlet extension axis. An inlet angle of at least 5°, preferably of at least 10°, more preferably of at least 15°, most preferably of at least 20°, may be provided between the inlet extension axis and the longitudinal axis of the inhaler article.

In other words, the air inlet may be tilted. The air inlet may be tilted away from the longitudinal axis of the inhaler article. A tilting of the air inlet may reduce leakage of powder through the air inlet. Additionally, tilting of the air inlet may create swirling vortices in the airflow which may improve the delivery of the powder to the consumer.

Similar to the tilting of the air outlet, the air inlet may be tilted to influence the airflow towards a user in a desired way.

The air outlet may be tilted towards a first sidewall of the inhaler article, preferably towards the first sidewall of the downstream compartment of the inhaler article. The air inlet may be tilted towards a second sidewall of the inhaler article, preferably towards a second sidewall of the downstream compartment of the inhaler article. The first sidewall may be arranged opposite the second sidewall.

In other words, the air outlet may be tilted to one side or first sidewall, while the air inlet may be tilted to the opposite side or second sidewall. This tilting to opposite sides of the air inlet and the air outlet may prevent powder from leaking. The reason for this is that no rectilinear line may be drawn from the air outlet to the air inlet when the air outlet is tilted to the opposite side of the air inlet. Preventing a rectilinear line between the air outlet and the air inlet may lead to reduced leakage when the device is turned during use.

The air outlet may be offset with respect to the longitudinal axis of the inhaler article.

In other words, the outlet extension axis may be parallel to and distanced from the longitudinal axis of the inhaler article. Providing the air outlet offset from the longitudinal axis of the inhaler article may prevent or reduce leakage of powder from the air outlet.

The air inlet may be offset with respect to the longitudinal axis of the inhaler article.

In other words, the inlet extension axis may be parallel to and distanced from the longitudinal axis of the inhaler article. Providing the air inlet offset from the longitudinal axis of the inhaler article may prevent or reduce leakage of powder from the air inlet.

The air inlet as well as the air outlet may each be offset with respect to the longitudinal axis of the inhaler article. In other words, the air outlet may be laterally offset in relation to the air inlet. The air inlet may be offset with respect to the longitudinal axis of the inhaler article in an opposite lateral direction than the air outlet. This arrangement may lead to preventing a rectilinear line between the air inlet and the outlet. This may reduce powder leakage.

The air outlet may comprise an outlet blocking wall. The outlet blocking wall may be arranged such that no rectilinear line can be drawn from the air outlet to the air inlet.

In case the air outlet is offset with respect to the longitudinal axis of the inhaler article, the outlet blocking wall may be arranged facing the longitudinal axis of the inhaler article. The outlet blocking wall may be tilted. The outlet blocking wall may be tilted away from the longitudinal axis of the inhaler article.

The air inlet may comprise an inlet blocking wall. The inlet blocking wall may be arranged such that no rectilinear line can be drawn from the air inlet to the air outlet.

In case the air inlet is offset with respect to the longitudinal axis of the inhaler article, the inlet blocking wall may be arranged facing the longitudinal axis of the inhaler article. The inlet blocking wall may be tilted. The inlet blocking wall may be tilted away from the longitudinal axis of the inhaler article.

An exit of the air inlet may be arranged distally distanced from the proximal end wall of the downstream compartment.

In case a consumer rotates the inhaler article during use or between uses, powder may be prevented from leaking out of the air inlet due to this arrangement of the exit of the air inlet.

The entrance to the air outlet may be arranged proximally distanced from the distal end wall of the downstream compartment such that a trough may be formed adjacent the distal end wall of the downstream compartment for excess powder.

The trough may be used to collect powder and thus prevent or reduce powder leakage. The air outlet may be circumscribed by the trough. The trough may be arranged coaxial with the air outlet. The air outlet may be arranged at the center of the trough. The air outlet may be raised in comparison to a base of the trough. The base of the trough may be formed by the distal end wall of the downstream compartment. The peripheral sidewalls of the trough may be formed by the sidewall of the downstream compartment. The inner sidewalls of the trough may be formed by the raised air outlet.

In case the air inlet is arranged distally distanced from the proximal end wall of the downstream compartment, a similar trough may be formed surrounding the air inlet. In case of rotation of the inhaler article, the powder may be held back from leaking out of the downstream compartment as the powder may be held in the trough surrounding the air inlet or in the trough surrounding the air outlet.

The trough may have a capacity of at least 10% of the powder contained in the article.

The downstream compartment may further comprise a central column extending from the proximal end wall of the downstream compartment in a distal direction.

The central column may prevent powder from falling through the air outlet in case the inhaler article is rotated. The central column may be arranged over the air outlet. The central column may be arranged distanced from the air outlet so that air can laterally flow into the air outlet between the air outlet and the central column. The central column may be arranged hanging over the air outlet. The central column may be mounted to our attached at the proximal end wall of the downstream compartment. The air inlet may be arranged next to the central column in the proximal end wall of the downstream compartment.

The proximal end wall of the downstream compartment may be a separating wall between the downstream compartment at the upstream compartment. The proximal end wall of the downstream compartment may be a distal end wall at the upstream compartment. The proximal end wall of the downstream compartment may be arranged between the downstream compartment at the upstream compartment. The proximal end wall of the downstream compartment may separate the inhaler article into similar sized compartments. These similar sized compartments may be the downstream compartment and the upstream compartment.

The proximal end wall of the downstream compartment may comprise an aperture. The air inlet may form the aperture. The air inlet may enable air to be drawn from the upstream compartment to the downstream compartment. The proximal end wall of the downstream compartment comprising the air inlet may create a separation between the upstream compartment and the downstream compartment. The air inlet may allow powder from the capsule arranged in the upstream compartment to be drawn into the downstream compartment.

The central column may be solid. This may prevent powder to fall through the central column. Alternatively, the central column may be hollow. In this case, however, the sidewalls as well as the distal end of the central column are preferably closed. The proximal end of the central column may be attached to the proximal end wall of the downstream compartment and therefore also closed.

The outer diameter of the central column may be larger than the inner diameter of the air outlet.

This may efficiently prevent powder from reaching the air outlet and leaking out of the air outlet in the absence of a draw of the user.

A distal end of the central column may be conical.

This may increase the amount of powder being drawn through the air outlet in case of a draw of a user.

The central column may be connected with the air outlet.

This arrangement may close the access to the air outlet in the area of the connection between the central column and the air outlet. It can thus be defined where the air can flow into the air outlet and it can be prevented that air flows into the air outlet from all lateral directions. This arrangement may be particularly beneficial if one or both of the air inlet and the air outlet are tilted. This arrangement may also be particularly beneficial if one or both of the air inlet and the air outlet are offset with respect to the longitudinal axis of the inhaler article. In all of these cases, the side of the air outlet facing the longitudinal axis of the aerosol generating article may be closed by the connection between the central column and the air outlet. The side of the air outlet facing the longitudinal axis of the aerosol generating article preferably is the side of the air outlet facing the air inlet.

The air outlet may have a laterally facing entrance. The laterally facing entrance may be arranged in a direction opposite to the direction towards the longitudinal axis of the inhaler article. In other words, the laterally facing entrance may face the sidewall of the inhaler article. Again in other words, the laterally facing entrance may face outwards.

The part of the central column connecting the central column with the air outlet may be chamfered.

The disclosure further relates to an inhaler article holder with a cavity configured for receiving an inhaler article as described herein. The inhaler article holder may comprise a piercing element for piercing the capsule.

The piercing element may be a pin. The piercing element may be slidable. The piercing element may reach into the cavity. The piercing element may be arranged retractable from the cavity. The piercing element may be arranged to slide into the cavity for piercing the capsule of an inhaler article arranged in the cavity. The piercing element may be arranged retractable from the cavity after piercing of the capsule of the inhaler article. The piercing element may be arranged centrally aligned within the cavity. The piercing element may be arranged along the longitudinal axis of the inhaler article. Alternatively, the piercing element may be arranged offset with respect to the longitudinal axis of the inhaler article. The longitudinal axis of the inhaler article may be identical to the longitudinal axis of the inhaler article holder.

The piercing element may be arranged at the base of the cavity. The piercing element may be arranged in an aperture at the base of the cavity. The piercing element may be arranged retractable through the aperture at base of the cavity.

The inhaler article holder may be configured to receive the inhaler article. The inhaler article holder may be configured to be used with a multitude of inhaler articles. After depletion of the inhaler article, a fresh inhaler article may be received in the cavity of the inhaler article holder.

The disclosure further relates to an inhaler system comprising an inhaler article as described herein and an inhaler article holder as described herein.

During a draw of the user, dry powder particles may exit the capsule through an aperture in the capsule. The dry powder particles may be entrained in the inhalation air flow to the consumer. The aperture in the capsule may be created by the piercing element. Before use, the capsule may be hermetically sealed.

Inhalable powders of the capsule of the inhaler article may include various active agents. The active agent may comprise an alkaloid such as nicotine or anatabine or anabasine, for example. Preferably, the active agent comprises solid salt of an alkaloid, such as a nicotine salt.

The amount of active agent may be selected based on the desired or intended use of the inhalable dry powder. For example, the amount of active agent may be between 0.5 wt-% and 10 wt-% of the total weight of the dry powder particles. The dry powder particles may comprises 0.5 wt-% or more, 1 wt-% or more, 2 wt-% or more, or 3 wt-% or more of the active agent, and 12 wt-% or less, 10 wt-% or less, 9 wt-% or less, 8 wt-% or less, or 7 wt-% or less, of the active agent, or from 0.5 wt-% to 10 wt-%, from 1 wt-% to 8 wt-%, from 1.5 wt-% to 6 wt-%, or from 2 wt-% to 5 wt-of the active agent.

The dry powder particles may comprises 0.5 wt-% or more, 1 wt-% or more, 2 wt-% or more, or 3 wt-% or more of nicotine, and 12 wt-% or less, 10 wt-% or less, 9 wt-% or less, 8 wt-% or less, or 7 wt-% or less, of nicotine, or from 0.5 wt-% to 10 wt-%, from 1 wt-% to 8 wt-%, from 1.5 wt-% to 6 wt-%, or from 2 wt-% to 5 wt-% nicotine.

The amount of active agent may also be selected on a per-dose basis. The inhalable powder may be packaged in a single dose form or in a multiple dose form. For example, the inhalable powder may comprise 0.5 mg or more, 1 mg or more, 2 mg or more, or 5 mg or more of the active agent per dose. The inhalable powder may comprise 500 mg or less, 200 mg or less, 100 mg or less, 50 mg or less, 20 mg or less, or 10 mg or less of the active agent per dose. In some embodiments, the inhalable powder comprises from 0.01 to 10 mg of anatabine or nicotine or anabasine per dose, 0.05 to 5 mg anatabine or nicotine or anabasine per dose, or 0.1 to 1 mg of anatabine or nicotine or anabasine per dose.

In embodiments, the capsule contains from 1 to 20 doses. In embodiments, the capsule contains from 1 to 10 doses. In embodiments the capsule contains from 10 to 20 doses.In embodiments, the capsule contains 1 dose. In embodiments, the capsule contains 2 doses. In embodiments, the capsule contains 3 doses. In embodiments, the capsule contains 4 doses. In embodiments, the capsule contains 5 doses. In embodiments, the capsule contains 6 doses. In embodiments, the capsule contains 7 doses. In embodiments, the capsule contains 8 doses. In embodiments, the capsule contains 9 doses. In embodiments, the capsule contains 10 doses. In embodiments, the capsule contains 11 doses. In embodiments, the capsule contains 12 doses. In embodiments, the capsule contains 13 doses. In embodiments, the capsule contains 14 doses. In embodiments, the capsule contains 15 doses. In embodiments, the capsule contains 16 doses. In embodiments, the capsule contains 17 doses. In embodiments, the capsule contains 18 doses. In embodiments, the capsule contains 19 doses. In embodiments, the capsule contains 20 doses.

The dry powder particles may have a particle size of 20 µm or less, 10 µm or less, or 5 µm or less, or 0.1 µm or greater, 0.2 µm or greater, or 0.5 µm or greater, or ranging from 0.5 µm to 10 µm, or from 0.75 µm to 5 µm, or from 1 µm to 5 µm, or from 1 µm to 3 µm, or from 1.5 µm to 2.5 µm. The desired particle size range may be achieved by spray drying, milling, sieving, or a combination thereof.

The dry powder particles may be further mixed with a second population of particles to form a powder system. Preferably, the second population of particles have a different particle size or larger particle size than the dry powder particles. For example, the second population of particles may have a particle size of about 20 µm or greater, or about 50 µm or greater, 200 µm or smaller, 150 µm or smaller, or in a range from 50 µm to 200 µm, or from 50 µm to 150 µm. The second population of particles may have any useful size distribution for inhalation delivery selectively into the mouth or buccal cavity of a user. The larger second population of flavourant particles may assist in delivery of the dry powder particles to the inhalation airflow to the user.

The dry powder particles and second population of particles may be combined in any useful relative amount so that the second population of particles are detected by the user when consumed with the dry powder particles. Preferably, the dry powder particles and second population of particles form at least about 90 wt-% or at least about 95 wt-% or at least about 99 wt-% or 100 wt-% of the total weight of the powder system.

The dry powder particles may be mixed with a second population of flavourant particles to form a powder system. Preferably, the second population of flavourant particles have a different particle size or larger particle size than the dry powder particles. For example, the flavor particles may have a particle size of about 20 µm or greater, or about 50 µm or greater, 200 µm or smaller, 150 µm or smaller, or in a range from 50 µm to 200 µm, or from 50 µm to 150 µm. The second population of flavourant particles may have any useful size distribution for inhalation delivery selectively into the mouth or buccal cavity of a user. The larger second population of flavourant particles may assist in delivery of the dry powder particles to the inhalation airflow to the user.

The dry powder particles and second population of flavourant particles may be combined in any useful relative amount so that the second population of flavourant particles are detected by the user when consumed with the dry powder particles. Preferably, the dry powder particles and second population of flavourant particles form at least about 90 wt-% or at least about 95 wt-% or at least about 99 wt-% or 100 wt-% of the total weight of the powder system.

The dry powder particles or powder system may be provided in a suitable dosage form. For example, the dry powder particles or powder system may be provided in a capsule. The dosage form (for example, capsule) may be configured for use in a suitable inhaler. For example, the capsule may be utilized in an inhaler device having a capsule cavity. Air flow management through a capsule cavity of the inhaler device may cause a capsule contained therein to rotate during inhalation and consumption. The capsule may contain dry powder particles or powder system.

The term "particle size" is used here to refer to the mass median aerodynamic diameter (MMAD) of the particle or set of particles, unless otherwise stated. Such values are based on the distribution of the aerodynamic particle diameters defined as the diameter of a sphere with a density of 1 gm/cm³ that has the same aerodynamic behavior as the particle which is being characterized.

In particular, for a powder system reference is commonly made to the mass median aerodynamic diameter (MMAD), one of the metrics most widely adopted as a single number descriptor of aerodynamic particle-size distribution. The MMAD is a statistically derived figure for a particle sample: by way of example, an MMAD of 5 micrometres means that 50 percent of the total sample mass will be present in particles having aerodynamic diameters of less than 5 micrometres, and that the remaining 50 percent of the total sample mass will be present in particles having an aerodynamic diameter greater than 5 micrometres. In the context of the present invention, when describing a powder system, the term "particle size" preferably refers to the MMAD of the powder system.

The MMAD of a powder system is preferably measured with a cascade impactor. Cascade impactors are instruments which have been extensively used for sampling and separating airborne particles for determining the aerodynamic size classification of aerosol particles. In practice, cascade impactors separate an incoming sample into discrete fractions on the basis of particle inertia, which is a function of particle size, density and velocity. A cascade impactor typically comprises a series of stages, each of which comprises a plate with a specific nozzle arrangement and a collection surface. As nozzle size and total nozzle area both decrease with increasing stage number, the velocity of the sample-laden air increases as it proceeds through the instrument. At each stage, particles with sufficient inertia break free from the prevailing air stream to impact on the collection surface. Therefore, at any given flow rate, each stage is associated with a cut-off diameter, a figure that defines the size of particles collected. With increasing stage number, velocity increases and so stage cut-off diameter decreases. Thus, the cut-off diameter associated with a given stage is a function of the airflow rate used for testing. To reflect in-use performance, nebulisers are routinely tested at 15 L/min and dry powder inhalers may be tested at flow rates up to 100 L/min.

Preferably, in the context of the present invention, the MMAD of a powder system is measured with a Next Generation Impactor (NGI) 170 (available from Copley Scientific AG). The NGI is a high performance, precision, particle classifying cascade impactor having seven stages plus a Micro-Orifice Collector (MOC). Characteristics and operation principle of a NGI are described, for example, in Marple et al., Journal of Aerosol Medicine - Volume 16, Number 3 (2003). More preferably, measurements are carried out at 20 ±3 degrees Celsius and relative humidity of 35 ± 5 percent.

A dry powder formulation typically contains less than or equal to about 15 percent by weight moisture, preferably less than or equal to about 10 percent moisture, even more preferably less than or equal to about 6 percent by weight moisture. Most preferably a dry powder formulation contains less than or equal to about 5 percent by weight moisture or even less than or equal to about 3 percent by weight moisture or even less than or equal to about 1 percent by weight moisture.

All values reported as a percentage are presumed to be weight percent based on the total weight.

The disclosure will be further described, by way of example only, with reference to the accompanying drawings in which:
Figs. 1A and 1B show an inhaler article;
Figs. 2A, 2B and 2C show rotation of the inhaler article;
Figs. 3A and 3B show an embodiment of the inhaler article with a central column in a downstream compartment;
Figs. 4A, 4B and 4C show rotation of the inhaler article having a central column;
Fig. 5 shows a variation of the central column of the inhaler article.

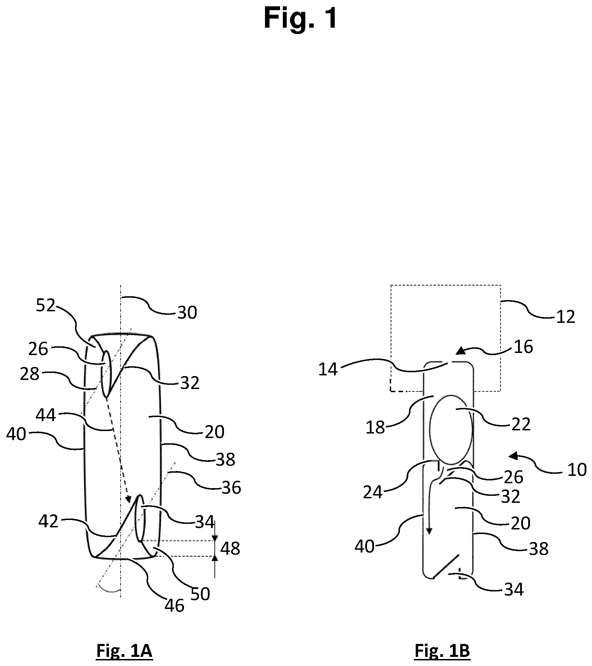

Figure 1B shows an inhaler article 10 and schematically an inhaler article holder 12. The inhaler article 10 is received in a cavity of the inhaler article holder 12.

The inhaler article 10 comprises a proximal opening 14 arranged at a proximal end 16 of the inhaler article 10. The proximal opening 14 is received in the cavity of the inhaler article holder 12. Air can be drawn into the inhaler article 10 through the proximal opening 14. The proximal opening 14 may further enable a piercing element of the inhaler article holder 12 to be inserted into the inhaler article 10.

The inhaler article 10 comprises an upstream compartment 18 at the downstream compartment 20. A capsule 22 containing an inhalable powder is arranged in the upstream compartment 18. The capsule 22 may be pierced by the insertion of the piercing element of the inhaler article holder 12 into the upstream compartment 18 of the inhaler article 10.

Between the upstream compartment 18 of the inhaler article 10 and the downstream compartment 20 of the inhaler article 10, a separation wall 24 is arranged. The separation wall 24 is the proximal wall of the downstream compartment 20 and the distal wall of the upstream compartment 18. The separation wall 24 prevents the capsule 22 from entering the downstream compartment 20. In other words, the separation wall 24 holds the capsule 22 within the upstream compartment 18.

An air inlet 26 is formed within the separation wall 24. The air inlet 26 allows air to be drawn from the upstream compartment 18 into the downstream compartment 20 as indicated by the arrow in Figure 1B. The air inlet 26 has an arrangement so as to prevent or reduce powder leakage in case the inhaler article 10 is rotated during use or between uses.

As shown in Figure 1A, an extension axis 28 of the air inlet 26 is tilted with respect to the longitudinal axis 30 of the inhaler article 10. Further, the air inlet 26 comprises an air inlet blocking wall 32 that faces the longitudinal axis 30 of the inhaler article 10. The air inlet blocking wall 32 together with the tilted configuration of the air inlet 26 prevents powder leakage from the air inlet 26 towards an air outlet 34 of the downstream compartment 20.

The air outlet 34 of the downstream compartment 20 also is tilted with respect to the longitudinal axis 30 of the inhaler article 10 similar to the tilting of the air inlet 26. As shown in Figure 1A, the air outlet 34 also has an extension axis 36 that is tilted with respect to the longitudinal axis 30 of the inhaler article 10. The air outlet 34 is tilted to an opposite direction than the tilting of the air inlet 26. As can be seen in Figure 1A, the air outlet 34 is tilted towards a first sidewall 38 of the downstream compartment 20, while the air inlet 26 is tilted towards an opposite second sidewall 40 of the downstream compartment 20. Further, the air outlet 34 comprises an air outlet blocking wall 42 facing the longitudinal axis 30 of the inhaler article 10.

The opposite tilting of the proximal opening 14 and the air outlet 34 together with the air inlet blocking wall 32 and the air outlet blocking wall 42 prevents a rectilinear line 44 being able to be drawn between the air inlet 26 and the air outlet 34.

Figure 1 additionally shows a raising of the air outlet 34 from a distal end wall 46 of the downstream compartment 20. In other words, a distance 48 is provided between the air outlet 34 and the distal end wall 46 of the downstream compartment 20. This distance 48 creates an air outlet trough 50 surrounding the air outlet 34 between the air outlet 34 and the distal end wall 46 of the downstream compartment 20. Powder that would conventionally leak from the air outlet 34 can be prevented from leaking out of the air outlet 34, since the powder falls into the air outlet trough 50.

Similarly, the air inlet 26 is arranged distally distanced from the separation wall 24 to create an air inlet trough 52 surrounding the air inlet 26.

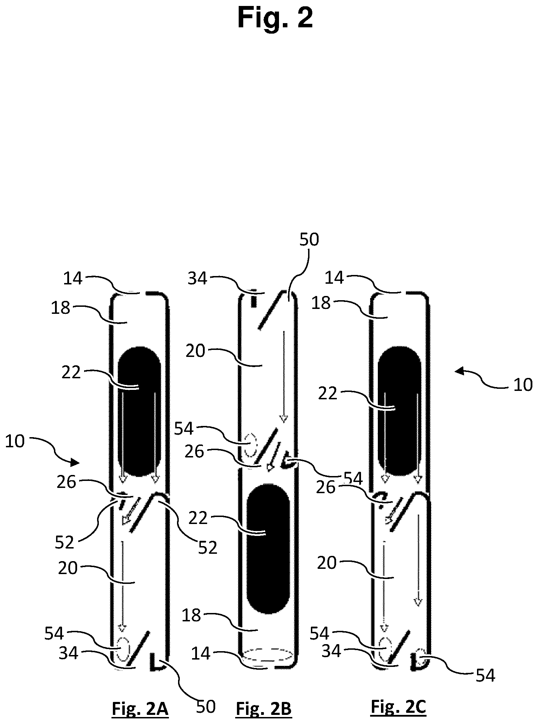

Figure 2 shows the prevention of leakage of powder 54 in case the inhaler article 10 is rotated during use or between uses. In Figure 2A, powder 54 leaking from the capsule 22 that is not drawn out of the air outlet 34 during a normal consumption process is prevented from leaking out of the air outlet 34. This leak powder 54 falls next to the air outlet 34 into the air outlet trough 50 formed between the distal end wall 46 of the downstream compartment 20 and the raised air outlet 34.

Figure 2B shows the movement of the powder 54 in case the inhaler article 10 is rotated by 180°. In this case, the powder 54 falls, inside of the downstream compartment 20, towards the upstream compartment 18. However, the powder does not leak into the upstream compartment 18 due to the configuration of the air inlet 26 being similar to the configuration of the air outlet 34. Hence, the powder falls into the air inlet trough 52 surrounding the air inlet 26. Particularly due to the opposite orientation of the tilting of the air inlet 26 and the air outlet 34 and the air inlet blocking wall 32 as well as the air outlet blocking wall 42, the powder 54 is prevented from leaking from the downstream compartment 20.

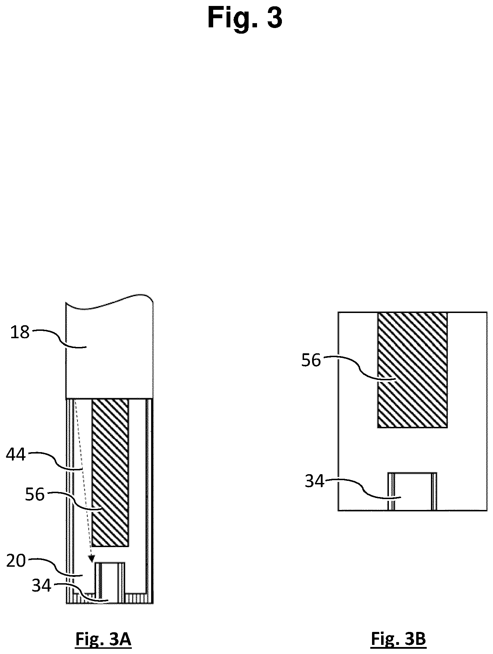

Figure 3C shows the subsequent rotation of the inhaler article 10 by again 180°. In other words, the inhaler article 10 is rotated back to the orientation shown in Figure 3A. The powder 54 that has been held next to the air inlet 26 in the downstream compartment 20 now falls again towards the air outlet 34 in the downstream compartment 20. However, the powder is prevented from leaking out of the air outlet 34 and instead falls into the air outlet trough 50 surrounding the air outlet 34.

Figure 3 shows an embodiment in which a central column 56 is provided extending from the separation wall 24 between the upstream compartment 18 and the downstream compartment 20 and extending in a distal direction towards the air outlet 34. The central column 56 has an outer diameter that is slightly larger than the inner diameter of the air outlet 34 such that no rectilinear line 44 can be drawn from the air inlet 26 to the air outlet 34. The air inlet 26 is not explicitly depicted in Figure 3. However, the air inlet 26 is arranged next to the central column 56 in the separation wall 24 as indicated by the rectilinear line 44 in Figure 3A. The air inlet 26 is thus arranged laterally offset with respect to the longitudinal axis 30 of the inhaler article 10.

Figure 3 B shows a more detailed view of the distal end of the central column 56 and the outer diameter of the central column 56 being larger than the inner diameter of the air outlet 34.

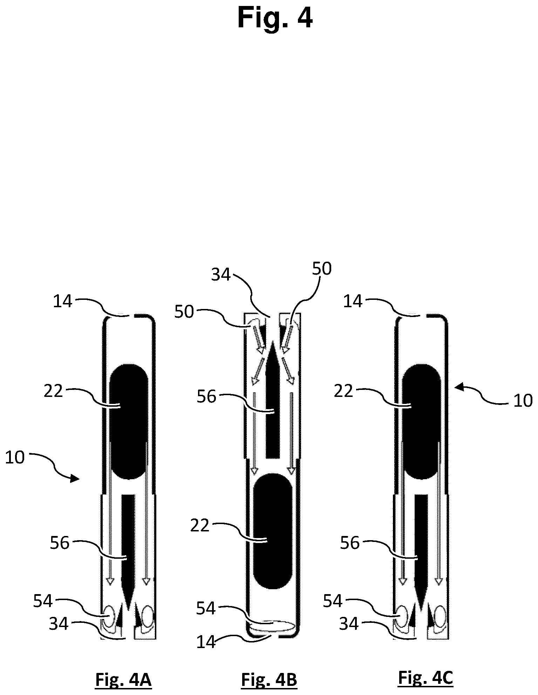

Figure 4 shows a rotation of the inhaler article 10 similar to the rotation shown in Figure 2. However, in this case the movement of the powder 54 is shown in case a central column 56 is provided as indicated in Figure 3. The central column 56 in the embodiment shown in Figure 4 is provided with a conical distal end to facilitate drawing of the powder 54 into the air outlet 34 during normal use. The air outlet 34 is raised as indicated in Figures 3 and 4 so that the air outlet trough 50 is formed next to the air outlet 34. Loose powder 54, instead of leaking out of the inhaler article 10, is deposited in the air outlet trough 50 during rotation of the inhaler article 10.

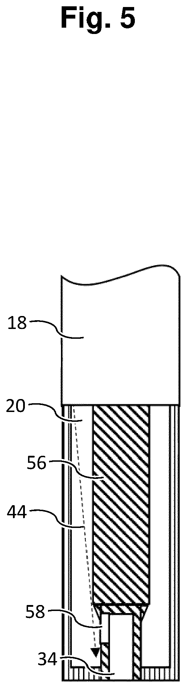

Figure 5 shows a variation of the central column 56 embodiment, in which the central column 56 is connected with the air outlet 34. A lateral opening 58 is provided in the central column 56 such that air can still reach the air outlet 34. The lateral opening 58 faces the sidewall of the downstream compartment 20 so that air can only get into the air outlet 34 through this laterally facing lateral opening 58.

## Claims

1. An inhaler article (10) comprising:
an upstream compartment (18), wherein a pierceable capsule (22) containing powder is arranged in the upstream compartment (18), and
a downstream compartment (20), wherein the downstream compartment (20) is fluidly connected with the upstream compartment (18) via an air inlet (26) arranged in a proximal end wall of the downstream compartment (20),
wherein the downstream compartment (20) comprises an air outlet (34) arranged in a distal portion of the downstream compartment (20), and wherein an entrance to the air outlet (34) is arranged proximally distanced from a distal end wall (46) of the downstream compartment (20),
wherein the air outlet (34) is angled towards a first sidewall of the inhaler article (10), wherein the air inlet (26) is angled towards a second sidewall of the inhaler article (10), wherein the first sidewall is arranged opposite the second sidewall.

2. The inhaler article (10) according to claim 1, wherein the air outlet (34) comprises an outlet extension axis (36), and wherein an outlet angle of at least 5°, preferably of at least 10°, more preferably of at least 15°, most preferably of at least 20°, is provided between the outlet extension axis (36) and a longitudinal axis (30) of the inhaler article (10).

3. The inhaler article (10) according to any preceding claim, wherein the air inlet (26) comprises an inlet extension axis (28), and wherein an inlet angle of at least 5°, preferably of at least 10°, more preferably of at least 15°, most preferably of at least 20°, is provided between the inlet extension axis (28) and a longitudinal axis (30) of the inhaler article (10).

4. The inhaler article (10) according to any preceding claim, wherein one or more of:
the air outlet (34) is offset with respect to a longitudinal axis (30) of the inhaler article (10),
the air inlet (26) is offset with respect to a longitudinal axis (30) of the inhaler article (10), and
the air outlet (34) is laterally offset in relation to the air inlet (26).

5. The inhaler article (10) according to any preceding claim, wherein the air outlet (34) comprises an outlet blocking wall (42), wherein the outlet blocking wall (42) is arranged such that no rectilinear line can be drawn from the air outlet (34) to the air inlet (26).

6. The inhaler article (10) according to any preceding claim, wherein the air inlet (26) comprises an inlet blocking wall (32), wherein the inlet blocking wall (32) is arranged such that no rectilinear line can be drawn from the air inlet (26) to the air outlet (34).

7. The inhaler article (10) according to any preceding claim, wherein an exit of the air inlet (26) is arranged distally distanced from the proximal end wall of the downstream compartment (20).

8. The inhaler article (10) according to any preceding claim, wherein the entrance to the air outlet (34) is arranged proximally distanced from the distal end wall (46) of the downstream compartment (20) such that a trough is formed adjacent the distal end wall (46) of the downstream compartment (20) for excess powder.

9. The inhaler article (10) according to any preceding claim, wherein the downstream compartment (20) further comprises a central column (56) extending from the proximal end wall of the downstream compartment (20) in a distal direction.

10. The inhaler article (10) according to the preceding claim, wherein one or more of:
the outer diameter of the central column (56) is larger than the inner diameter of the air outlet (34),
a distal end of the central column (56) is conical, and
the central column (56) is connected with the air outlet (34).

11. The inhaler article (10) according to the preceding claim, wherein the air outlet (34) has a laterally facing entrance.

12. The inhaler article (10) according to any of the two preceding claims, wherein the part of the central column (56) connecting the central column (56) with the air outlet (34) is chamfered.

13. An inhaler system comprising an inhaler article (10) according to any of claim 1 to 12 and an inhaler article holder (12) with a cavity configured for receiving the inhaler article (10), wherein the inhaler article holder (12) comprises a piercing element for piercing the capsule (22)

## Patentansprüche

1. Inhalatorartikel (10), aufweisend:
einer vorgelagerten Kammer (18), wobei in der vorgelagerten Kammer (18) eine durchstechbare Kapsel (22) angeordnet ist, die Pulver umfasst, und
eine nachgelagerte Kammer (20), wobei die nachgelagerte Kammer (20) über einen in einer proximalen Endwand der nachgelagerten Kammer (20) angeordneten Lufteinlass (26) mit der vorgelagerten Kammer (18) fluidisch verbunden ist,
wobei die nachgelagerte Kammer (20) einen Luftauslass (34) aufweist, der in einem distalen Abschnitt der nachgelagerten Kammer (20) angeordnet ist, und wobei ein Eingang zu dem Luftauslass (34) in proximaler Richtung in einem Abstand von einer distalen Endwand (46) der nachgelagerten Kammer (20) angeordnet ist,
wobei der Luftauslass (34) in Richtung einer ersten Seitenwand des Inhalatorartikels (10) abgewinkelt ist, wobei der Lufteinlass (26) in Richtung einer zweiten Seitenwand des Inhalatorartikels (10) abgewinkelt ist, wobei die erste Seitenwand der zweiten Seitenwand gegenüberliegend angeordnet ist.

2. Inhalatorartikel (10) nach Anspruch 1, wobei der Luftauslass (34) eine Auslassverlängerungsachse (36) umfasst, und wobei zwischen der Auslassverlängerungsachse (36) und einer Längsachse (30) des Inhalatorartikels (10) ein Auslasswinkel von wenigstens 5°, bevorzugt von wenigstens 10°, noch bevorzugter von wenigstens 15°, am meisten bevorzugt von wenigstens 20°, vorgesehen ist.

3. Inhalatorartikel (10) nach einem beliebigen vorhergehenden Anspruch, wobei der Lufteinlass (26) eine Einlassverlängerungsachse (28) umfasst, und wobei zwischen der Einlassverlängerungsachse (28) und einer Längsachse (30) des Inhalatorartikels (10) ein Einlasswinkel von wenigstens 5°, bevorzugt von wenigstens 10°, noch bevorzugter von wenigstens 15°, am meisten bevorzugt von wenigstens 20°, vorgesehen ist.

4. Inhalatorartikel (10) nach einem beliebigen vorhergehenden Anspruch, wobei eines oder mehrere zutreffen von:
der Luftauslass (34) ist in Bezug auf eine Längsachse (30) des Inhalatorartikels (10) versetzt,
der Lufteinlass (26) ist in Bezug auf eine Längsachse (30) des Inhalatorartikels (10) versetzt, und
der Luftauslass (34) ist in Bezug auf den Lufteinlass (26) seitlich versetzt.

5. Inhalatorartikel (10) nach einem beliebigen vorhergehenden Anspruch, wobei der Luftauslass (34) eine Auslass-Sperrwand (42) aufweist, wobei die Auslass-Sperrwand (42) so angeordnet ist, dass keine geradlinige Linie von dem Luftauslass (34) zu dem Lufteinlass (26) gezogen werden kann.

6. Inhalatorartikel (10) nach einem beliebigen vorhergehenden Anspruch, wobei der Lufteinlass (26) eine Einlass-Sperrwand (32) aufweist, wobei die Einlass-Sperrwand (32) so angeordnet ist, dass keine geradlinige Linie von dem Lufteinlass (26) zu dem Luftauslass (34) gezogen werden kann.

7. Inhalatorartikel (10) nach einem beliebigen vorhergehenden Anspruch, wobei ein Auslass des Lufteinlasses (26) distal von der proximalen Endwand der nachgelagerten Kammer (20) beabstandet angeordnet ist.

8. Inhalatorartikel (10) nach einem beliebigen vorhergehenden Anspruch, wobei der Eingang zu dem Luftauslass (34) proximal von der distalen Endwand (46) der nachgelagerten Kammer (20) beabstandet angeordnet ist, sodass angrenzend an die distale Endwand (46) der nachgelagerten Kammer (20) eine Mulde für überschüssiges Pulver gebildet wird.

9. Inhalatorartikel (10) nach einem beliebigen vorhergehenden Anspruch, wobei die nachgelagerte Kammer (20) ferner eine zentrale Säule (56) umfasst, die sich von der proximalen Endwand der nachgelagerten Kammer (20) in einer distalen Richtung erstreckt.

10. Inhalatorartikel (10) nach dem vorhergehenden Anspruch, wobei eines oder mehrere zutreffen von:
der Außendurchmesser der zentralen Säule (56) ist größer als der Innendurchmesser des Luftauslasses (34),
ein distales Ende der zentralen Säule (56) ist konisch, und
die zentrale Säule (56) ist mit dem Luftauslass (34) verbunden.

11. Inhalatorartikel (10) nach dem vorhergehenden Anspruch, wobei der Luftauslass (34) einen seitlich ausgerichteten Eingang aufweist.

12. Inhalatorartikel (10) nach einem der zwei vorhergehenden Ansprüche, wobei der Teil der zentralen Säule (56), der die zentrale Säule (56) mit dem Luftauslass (34) verbindet, abgeschrägt ist.

13. Inhalatorsystem, umfassend einen Inhalatorartikel (10) nach einem beliebigen von Anspruch 1 bis 12 und einen Inhalatorartikelhalter (12) mit einem Hohlraum, der zum Aufnehmen des Inhalatorartikels (10) eingerichtet ist, wobei der Inhalatorartikelhalter (12) ein Durchstechelement zum Durchstechen der Kapsel (22) umfasst.

## Revendications

1. Article inhalateur (10) comprenant :
un compartiment amont (18), dans lequel une capsule perçable (22) contenant de la poudre est agencée dans le compartiment amont (18), et
un compartiment aval (20), dans lequel le compartiment aval (20) est raccordé fluidiquement au compartiment amont (18) par l'intermédiaire d'une entrée d'air (26) agencée dans une paroi d'extrémité proximale du compartiment aval (20),
dans lequel le compartiment aval (20) comprend une sortie d'air (34) agencée dans une portion distale du compartiment aval (20), et dans lequel une arrivée vers la sortie d'air (34) est agencée proximalement à distance d'une paroi d'extrémité distale (46) du compartiment aval (20),
dans lequel la sortie d'air (34) est inclinée vers une première paroi latérale de l'article inhalateur (10), dans lequel l'entrée d'air (26) est inclinée vers une deuxième paroi latérale de l'article inhalateur (10), dans lequel la première paroi latérale est agencée à l'opposé de la deuxième paroi latérale.

2. Article inhalateur (10) selon la revendication 1, dans lequel la sortie d'air (34) comprend un axe d'extension de sortie (36), et dans lequel un angle de sortie d'au moins 5°, de préférence d'au moins 10°, de manière davantage préférée d'au moins 15°, de manière préférée entre toutes d'au moins 20 °, est prévu entre l'axe d'extension de sortie (36) et un axe longitudinal (30) de l'article inhalateur (10).

3. Article inhalateur (10) selon une quelconque revendication précédente, dans lequel l'entrée d'air (26) comprend un axe d'extension d'entrée (28), et dans lequel un angle d'entrée d'au moins 5°, de préférence d'au moins 10°, de manière davantage préférée d'au moins 15°, de manière préférée entre toutes d'au moins 20°, est prévu entre l'axe d'extension d'entrée (28) et un axe longitudinal (30) de l'article inhalateur (10).

4. Article inhalateur (10) selon une quelconque revendication précédente, dans lequel un ou plusieurs parmi :
la sortie d'air (34) est décalée par rapport à l'axe longitudinal (30) de l'article inhalateur (10),
l'entrée d'air (26) est décalée par rapport à l'axe longitudinal (30) de l'article inhalateur (10), et
la sortie d'air (34) est décalée latéralement par rapport à l'entrée d'air (26).

5. Article inhalateur (10) selon une quelconque revendication précédente, dans lequel la sortie d'air (34) comprend une paroi de blocage de sortie (42), dans lequel la paroi de blocage de sortie (42) est agencée de telle sorte qu'aucune ligne rectiligne ne peut être tracée depuis la sortie d'air (34) vers l'entrée d'air (26).

6. Article inhalateur (10) selon une quelconque revendication précédente, dans lequel l'entrée d'air (26) comprend une paroi de blocage d'entrée (32), dans lequel la paroi de blocage d'entrée (32) est agencée de telle sorte qu'aucune ligne rectiligne ne peut être tracée depuis l'entrée d'air (26) vers la sortie d'air (34).

7. Article inhalateur (10) selon une quelconque revendication précédente, dans lequel une sortie de l'entrée d'air (26) est agencée distalement à distance de la paroi d'extrémité proximale du compartiment aval (20).

8. Article inhalateur (10) selon une quelconque revendication précédente, dans lequel l'arrivée de la sortie d'air (34) est agencée proximalement à distance de la paroi d'extrémité distale (46) du compartiment aval (20) de telle sorte qu'une goulotte est formée de manière adjacente à la paroi d'extrémité distale (46) du compartiment aval (20) pour la poudre en excès.

9. Article inhalateur (10) selon une quelconque revendication précédente, dans lequel le compartiment aval (20) comprend en outre une colonne centrale (56) s'étendant depuis la paroi d'extrémité proximale du compartiment aval (20) dans une direction distale.

10. Article inhalateur (10) selon la revendication précédente, dans lequel un ou plusieurs parmi :
le diamètre extérieur de la colonne centrale (56) est plus grand que le diamètre intérieur de la sortie d'air (34),
une extrémité distale de la colonne centrale (56) est conique, et
la colonne centrale (56) est raccordée à la sortie d'air (34).

11. Article inhalateur (10) selon la revendication précédente, dans lequel la sortie d'air (34) a une arrivée orientée latéralement.

12. Article inhalateur (10) selon l'une quelconque des deux revendications précédentes, dans lequel la partie de la colonne centrale (56) raccordant la colonne centrale (56) à la sortie d'air (34) est chanfreinée.

13. Système inhalateur comprenant un article inhalateur (10) selon l'une quelconque des revendications 1 à 12 et un support d'article inhalateur (12) avec une cavité configurée pour recevoir l'article inhalateur (10), dans lequel le support d'article inhalateur (12) comprend un élément de perçage pour percer la capsule (22).
